# EUROPEAN PATENT APPLICATION

(11) **EP 2 548 614 A1**
(43) Date of publication of application: **23.01.2013**
(21) Application number: 11755844.5
(22) Date of filing: 07.03.2011
(51) Int. Cl.: A61N 5/06, A61B 1/00, A61B 19/00

(54) **CALCULATION DEVICE AND CALCULATION METHOD**

(30) Priority: 15.03.2010 JP 2010058386
(71) Applicant: Sony Corporation, Tokyo 108-0075 (JP); Keio University, Tokyo 108-8345 (JP)
(72) Inventor: TAMAMURA, Koshi, Tokyo 108-0075 (JP); HAKOMORI, Shiho, Tokyo 108-0075 (JP); YAMAGUCHI, Takashi, Tokyo 108-0075 (JP); ARAI, Tsunenori, Yokohama-shi Kanagawa (JP); ITO, Arisa, Yokohama-shi Kanagawa (JP)
(74) Representative: Müller - Hoffmann & Partner
(86) International application number: PCT/JP2011/001326
(87) International publication number: WO 2011/114651

(57) **Abstract**

[Object] To provide a calculation apparatus and a calculation method capable of calculating pharmaceutical concentration in a tissue in real time.

[Solving Means] A photodynamic therapy apparatus 1 as a calculation apparatus is an apparatus for irradiating a tissue having absorbed photo-sensitive pharmaceutical, the photo-sensitive pharmaceutical absorbing an excitation light and emitting fluorescence, with the excitation light emitted from a tip portion of a laser catheter 300, including a connector 210, a light source 110, and a light detection unit 130. The laser catheter 300 is capable of being attached/detached to/from the connector 210. The light source 110 outputs the excitation light to the laser catheter 300 via the connector 210. The light detection unit 130 detects intensity of the fluorescence, the fluorescence being entered from the laser catheter 300 via the connector 210, to calculate concentration of the photo-sensitive pharmaceutical in a tissue, the tip portion of the laser catheter 300 contacting the tissue.

## Description

### Technical Field

The present invention relates to a calculation apparatus and a calculation method that calculate pharmaceutical concentration in a tissue.

### Background Art

Atrial fibrillation is known as a kind of tachyarrhythmia. A hyperexcited site, which generates an electrical pulse, appears in the vicinity of a root portion, in which a pulmonary vein and a left atrium are connected, and the left atrium minutely vibrates and contracts because of the electrical pulse stimulation, to thereby cause an atrial fibrillation.

As an atrial fibrillation therapeutic method, the inventors have been proposed application of photodynamic therapy (hereinafter, referred to as "PDT".) (for example, see Patent Document 1.). In PDT, a cardiac-muscle tissue, which has absorbed photo-sensitive pharmaceutical, is irradiated with an excitation light by using a laser catheter, to thereby generate singlet oxygen. The singlet oxygen as a strong oxidizer insults a cardiac-muscle tissue, which surrounds the hyperexcited site, to thereby form an electric-conduction block, which blocks conduction of the electrical pulse from the hyperexcited site to the left atrium. As a result, an electric conduction between the hyperexcited site and the left atrium is blocked, and an abnormal vibration and contraction of the left atrium is inhibited.

Photo-sensitive pharmaceutical has a property of selectively accumulating in a certain tissue. In view of this, in general, after a predetermined time (for example, 8 to 48 hours) passes after photo-sensitive pharmaceutical is administered in a patient, when the state where the photo-sensitive pharmaceutical concentration is high in a therapy-target tissue and the photo-sensitive pharmaceutical concentration is low in other tissues and blood is established, that is, when the state where a so-called photo-sensitive pharmaceutical contrast is high is established, irradiation with the excitation light is started. Further, recently, PDT, in which the accumulating property of photo-sensitive pharmaceutical is not used and in which irradiation with the excitation light is started when photo-sensitive pharmaceutical is delivered to a therapy-target tissue by blood, is proposed.

Patent Document 1: WIPO Publication No. 2008/066126

### Disclosure of the Invention

### Problem to be solved by the Invention

In a therapy, in which pharmaceutical is used, it is important to monitor pharmaceutical concentration in a tissue to determine optimum therapeutic protocols.

In view of this, conventionally, as a method of monitoring pharmaceutical concentration in blood, a method of measuring absorbance of blood collected every predetermined time after pharmaceutical administration, and other methods are known. However, in this method, the plot number is limited because the collectable blood volume is limited, and in addition, it is not possible to measure the concentration in real time. Further, as a method of monitoring pharmaceutical concentration in a tissue, there is known a method in which part of carbon in pharmaceutical is transformed into isotope, the isotope is simultaneously administered, and pharmaceutical concentration in each tissue is measured based on a radiation quantity. However, this method involves radiation exposure problems, is not a less-invasive monitoring method, and is not realistic.

In view of the above-mentioned circumstances, an object of the present invention is to provide a calculation apparatus and a calculation method capable of calculating pharmaceutical concentration in a tissue in real time.

### Means for solving the Problem

To attain the above-mentioned object, a calculation apparatus according to an embodiment of the present invention is a calculation apparatus for irradiating a tissue having absorbed photo-sensitive pharmaceutical, the photo-sensitive pharmaceutical absorbing an excitation light and emitting fluorescence, with the excitation light emitted from a tip portion of a laser catheter, including a connector, a light source, and a detection unit.
Note that, in the specification, the term "tissue" may sometimes include blood.
The laser catheter is capable of being attached/detached to/from the connector.
The light source outputs the excitation light to the laser catheter via the connector.
The detection unit detects intensity of the fluorescence, the fluorescence being entered from the laser catheter via the connector, to calculate concentration of the photo-sensitive pharmaceutical in a tissue, the tip portion of the laser catheter contacting the tissue.

By detecting the intensity of the fluorescence entered from the laser catheter, it is possible to estimate concentration of the photo-sensitive pharmaceutical in a tissue, which the tip portion of the laser catheter contacts, in real time. Further, by using a laser catheter used for therapy, operability is improved.

The calculation apparatus may further include a calculation unit for calculating the concentration of the photo-sensitive pharmaceutical in the tissue, the tip portion of the laser catheter contacting the tissue, based on intensity of the detected fluorescence.

By detecting the intensity of the fluorescence entered from the laser catheter, it is possible to calculate concentration of the photo-sensitive pharmaceutical in a tissue, which the tip portion of the laser catheter contacts, in real time.

The calculation apparatus may further include a controller for outputting a signal to prompt to additionally administer the photo-sensitive pharmaceutical based on the calculated concentration.

As a result, it is possible to prompt a practitioner to additionally administer the photo-sensitive pharmaceutical in real time based on the intensity of the fluorescence entered from the laser catheter. Note that to "output a signal" means to output a display instruction including display information to a display unit, or to output a sound output instruction to a speaker unit.

The controller may calculate an excitation-light-irradiation protocol based on the calculated concentration, and output a calculation result.

As a result, it is possible to inform a practitioner of the excitation-light-irradiation protocol in real time, based on the intensity of the fluorescence entered from the laser catheter.

A calculation method according to an embodiment of the present invention includes irradiating a tissue having absorbed photo-sensitive pharmaceutical, the photo-sensitive pharmaceutical absorbing an excitation light and emitting fluorescence, with the excitation light emitted from a tip portion of a laser catheter.
The fluorescence corresponding to the irradiated excitation light is extracted via the laser catheter.
Concentration of the photo-sensitive pharmaceutical in a tissue, the tip portion of the laser catheter contacting the tissue, is calculated based on intensity of the extracted fluorescence.

By detecting the intensity of the fluorescence entered from the laser catheter, it is possible to estimate concentration of the photo-sensitive pharmaceutical in a tissue, which the tip portion of the laser catheter contacts, in real time.

The calculation method may further include outputting a signal to prompt to additionally administer the photo-sensitive pharmaceutical based on the calculated concentration.

As a result, it is possible to prompt a practitioner to additionally administer the photo-sensitive pharmaceutical in real time based on the intensity of the fluorescence entered from the laser catheter.

The calculation method may further include calculating an excitation-light-irradiation protocol based on the calculated concentration, and outputs a calculation result.

As a result, it is possible to inform a practitioner of the excitation-light-irradiation protocol in real time, based on the intensity of the fluorescence entered from the laser catheter.

A calculation method according to an embodiment of the present invention is a calculation method using photo-sensitive pharmaceutical absorbing an excitation light and emitting a fluorescence, a laser catheter capable of emitting the excitation light from a tip portion, and a calculation apparatus including a connector to/from which the laser catheter is capable of being attached/detached and a light source for outputting the excitation light to the laser catheter via the connector.
In a tissue, the photo-sensitive pharmaceutical is absorbed.
The tip portion of the laser catheter is led to the tissue having absorbed the photo-sensitive pharmaceutical, the laser catheter being attached to the connector.
The tissue having absorbed the photo-sensitive pharmaceutical is irradiated with the excitation light emitted from the tip portion of the laser catheter, the excitation light being output from the light source.
The fluorescence corresponding to the irradiated excitation light is extracted via the laser catheter.
Concentration of the photo-sensitive pharmaceutical in a tissue, the tip portion of the laser catheter contacting the tissue, is calculated based on intensity of the extracted fluorescence.

The calculation method may further include calculating an excitation-light-irradiation protocol based on the calculated concentration, and outputs a calculation result.

### Effect of the Invention

According to the present invention, it is possible to calculate pharmaceutical concentration in a tissue in real time.

### Brief Description of Drawings

[Fig. 1] A schematic diagram showing a PDT apparatus according to a first embodiment of the present invention.
[Fig. 2] A schematic diagram showing a laser catheter inserted in a heart.
[Fig. 3] A block diagram showing a PDT apparatus main body.
[Fig. 4] A sectional view showing the tip portion of the laser catheter.
[Fig. 5] A flowchart showing operations of the PDT apparatus.
[Fig. 6] A schematic diagram showing the laser catheter inserted in a left atrium.
[Fig. 7] A graph showing the temporal change of fluorescence intensity.
[Fig. 8] A graph showing the correlation between the fluorescence intensity and pharmaceutical concentration.
[Fig. 9] A graph showing the temporal change of the pharmaceutical concentration.
[Figs. 10] Schematic diagrams each showing a contact state of the laser catheter.
[Fig. 11] A graph showing the temporal change of the fluorescence intensity.
[Fig. 12] Another graph showing the temporal change of the fluorescence intensity.
[Fig. 13] A schematic diagram showing a movement track of the laser catheter.
[Fig. 14] A diagram showing the relation of ECG, intracardiac pressure, and coronary blood-flow volume, which is dominant in the blood-flow volume in a cardiac-muscle tissue.
[Fig. 15] A diagram showing the correlation between fluorescence intensity and R-wave when the laser catheter is in the upright-contact state.
[Fig. 16] A diagram showing the correlation between fluorescence intensity and R-wave when the laser catheter is in the slanting-contact state.
[Fig. 17] A block diagram showing an optical system, a detection unit, and the like of a second embodiment of the present invention.
[Figs. 18] Schematic diagrams each showing a contact state of a laser catheter in an intravascular lumen.
[Fig. 19] A graph showing the relation between wavelength and fluorescence intensity.

### Best Modes for Carrying Out the Invention

Hereinafter, embodiments of the present invention will be described with reference to the drawings. In the embodiments, the case where a photodynamic therapy apparatus (hereinafter referred to as "PDT apparatus".) is used as a calculate apparatus will be described.

### <First Embodiment>

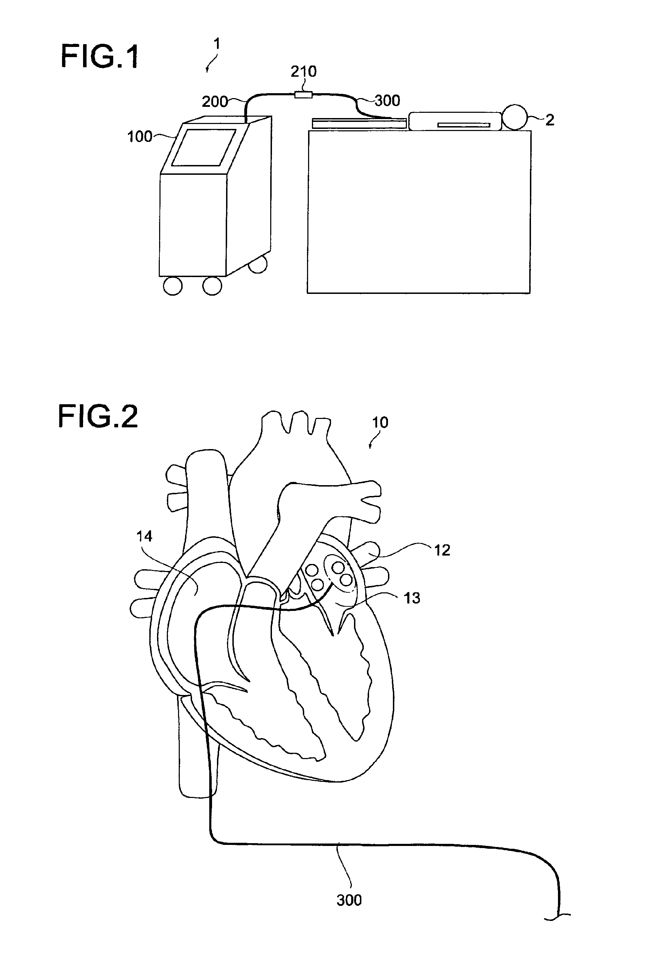

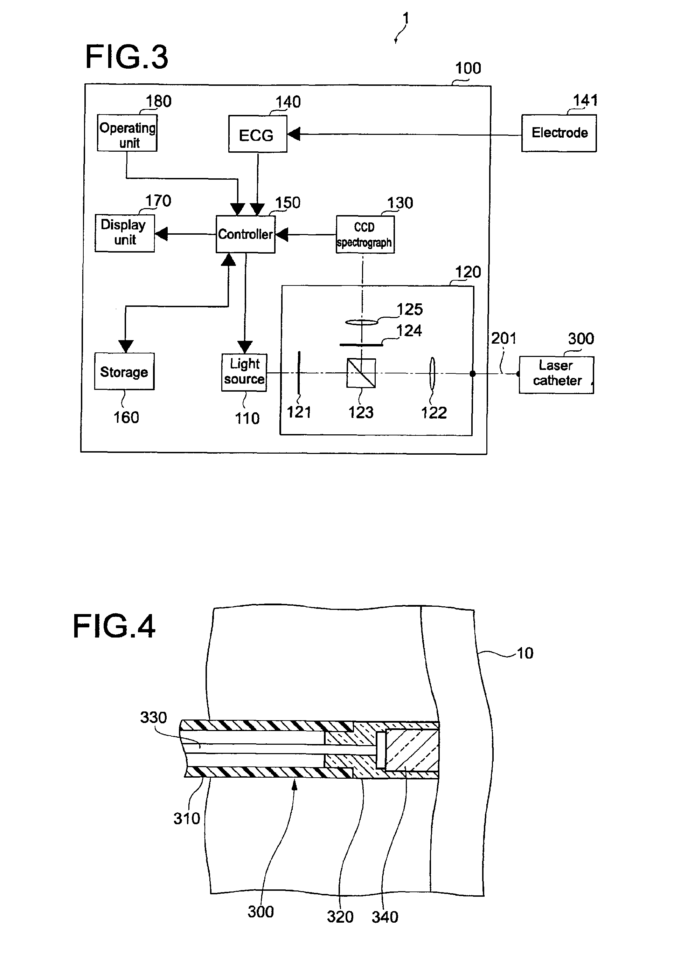

Fig. 1 is a schematic diagram showing a PDT apparatus according to a first embodiment of the present invention.
The PDT apparatus 1 includes a PDT apparatus main body 100, a tube 200 connected to the PDT apparatus main body 100, and a connector 210 provided on the end of the tube 200.
The tube 200 is a soft hollow tube, and is capable of transmitting light via an inner apparatus-attached optical fiber 201 (see Fig. 3.).
A laser catheter 300 is detachably connected to the connector 210.

Photo-sensitive pharmaceutical is administered to a patient 2. In the case of being administered by intravenous injection, the administered photo-sensitive pharmaceutical diffuses in the blood, and then a tissue such as a cardiac-muscle tissue absorbs the pharmaceutical. A dose of photo-sensitive pharmaceutical necessary for therapy may be administered at one time by intravenous injection, may be administered continuously by intravenous drip, may be administered at one time or continuously via the oral route, or may be administered locally. Photo-sensitive pharmaceutical is pharmaceutical that absorbs light having a certain wavelength, is photoexcited, and becomes fluorescent. For example, pharmaceutical called talaporfin sodium (Laserphyrin (registered trademark), Meiji Co., Ltd.) is employed. Because the Q-band absorption wavelength of this pharmaceutical is near 664 nm, an excitation light source for this pharmaceutical with, for example, 600 to 800 nm, preferably 660 to 680 nm, or more preferably 664 plus or minus 2 nm is used.

Fig. 2 is a schematic diagram showing a laser catheter inserted in a heart.
The laser catheter 300 is inserted in a right atrium 14 of a heart 10 via a femoral vein or a jugular vein of the patient 2. The laser catheter 300, which has reached the right atrium 14, penetrates a septum, and is led to a left atrium 13.

### [Configuration of PDT apparatus main body]

Fig. 3 is a block diagram showing the PDT apparatus main body.
The PDT apparatus main body 100 includes a light source 110, an optical system 120, a detection unit 130, an electrocardiograph 140, a controller 150, storage 160, a display unit 170, and an operating unit 180.

The light source 110 outputs an excitation light for photo-sensitive pharmaceutical. The wavelength of the light output by the light source 110 is the same as the Q-band absorption wavelength of the photo-sensitive pharmaceutical. For example, in the case where photo-sensitive pharmaceutical whose Q-band absorption wavelength is near 664 nm is used, a semiconductor laser with the emission wavelength of 600 to 800 nm, preferably 660 to 680 nm, or more preferably 664 plus or minus 2 nm is used as the light source 110. The excitation light output by the light source 110 enters the laser catheter 300 via the optical system 120.

The optical system 120 allows the excitation light, which is emitted from the light source 110, to enter the laser catheter 300, which is connected to the connector 210 via the apparatus-attached optical fiber 201. The optical system 120 extracts, from the laser catheter 300, fluorescence emitted from photo-sensitive pharmaceutical, which is irradiated with the excitation light, and allows the fluorescence to enter the detection unit 130. The optical system 120 includes a short pass filter 121, a first lens 122, a polarizing beam splitter (hereinafter referred to as "PBS".) 123, a long pass filter 124, and a second lens 125.
The short pass filter 121 is a short-wavelength transmission filter with a cuton wavelength of 670 nm, and cuts long-wavelength radiation. The excitation light from the light source 110 has the radiation component in the fluorescence observation wavelength range (long-wavelength side of peak wavelength). In view of this, the radiation component of the excitation light in the long-wavelength side is cut at the stage prior to collecting the light in the laser catheter 300. The excitation light, which has passed the short pass filter 121, enters the first lens 122.
The first lens 122 collects the excitation light, which has entered from the short pass filter 121, on one edge of the laser catheter 300. Further, the first lens 122 collects fluorescence from the tip portion of the laser catheter 300 on the PBS 123. Note that part of the excitation light from the light source 110 is reflected off an edge of the apparatus-attached optical fiber 201 at the PDT apparatus main body 100 side, off the inside of the connector 210, and off the tip portion of the laser catheter 300, and enters the PBS 123 as specular reflection light. The specular reflection light is noisy when detecting fluorescence.
By using polarization differences, the PBS 123 allows the specular reflection light, which has reflected off an edge of the optical fiber in the tube 200, out of the light entered from the first lens 122, to pass through, does not detect the specular reflection light, reflects fluorescence and the specular reflection light reflected off the other edges, and brings them to a detecting device. The fluorescence, which has passed the PBS 123, enters the long pass filter 124.
The long pass filter 124 causes the specular reflection light, which has reflected off the inside of the connector 210 and the tip portion of the laser catheter 300, out of the light entered from the PBS 123, not to pass through, allows only the fluorescence to pass through, and brings the fluorescence to the detecting device. The fluorescence, which has passed through the long pass filter 124, enters the second lens 125.
The second lens 125 collects the fluorescence, which has entered from the long pass filter 124, on the detection unit 130.

The detection unit 130 is, for example, a linear image sensor, and spectroscopically detects the fluorescence entered from the optical system 120. That is, the detection unit 130 detects the light having the excitation wavelength, and detects the fluorescence from the photo-sensitive pharmaceutical, which is a light having a wavelength longer than the excitation wavelength. The detection unit 130 outputs an electrical signal, which shows intensity of the detected fluorescence, to the controller 150.

An electrode pad 141 is connected to the electrocardiograph 140 via an electrode code (not shown). The electrocardiograph 140 obtains an electrocardiographic signal of the patient 2 via the electrode pad 141, which is attached to the patient 2, and via the electrode code, and supplies the obtained electrocardiographic signal to the controller 150.

The controller 150 controls the respective units of the PDT apparatus 1.
The controller 150 calculates fluorescence intensity based on the electrical signal obtained from the detection unit 130. The controller 150 calculates pharmaceutical concentration in the tissue or in the blood based on the calculated fluorescence intensity (pharmaceutical-concentration-monitoring operation). The controller 150 determines whether to additionally administer the pharmaceutical or not based on the calculated pharmaceutical concentration.
The controller 150 determines the contact state of the laser catheter 300 with respect to the tissue based on the electrical signal obtained from the detection unit 130 (contact-monitoring operation).
The controller 150 determines, based on change of the fluorescence intensity during excitation light irradiation, whether an abnormal situation such as a foreign substance or a breakage occurs or not, and determines the cytocidal effect (foreign-substance/breakage-monitoring operation, and cytocidal-effect-determining operation). The controller 150 controls the light source 110 to stop irradiating the excitation light based on determination results.
The controller 150 determines whether an electric-conduction block is formed or not based on an electrical signal obtained from the detection unit 130 and based on an electrocardiographic signal obtained from the electrocardiograph 140 (electric-conduction-block-formation determining operation).
The controller 150 outputs, to the display unit 170, display instructions to display the above-mentioned various calculation results, the above-mentioned various determination results, and various information.

The storage 160 is a nonvolatile memory, and is set in, for example, a flash memory, an HDD (Hard Disk Drive), or another solid memory. The controller 150 records, in the storage 160, temporal change of fluorescence intensity, in which information on fluorescence intensity obtained from the detection unit 130 is in relation with time information obtained from a timing measurement unit (not shown), which measures the elapsed time after a criterion time such as excitation-light-irradiation start time. The controller 150 records, in the storage 160, electrocardiograms in which information on an electrocardiographic signal obtained from the electrocardiograph 140 is in relation with time information.

The display unit 170 is a display device, which uses, for example, a liquid-crystal display device or the like. When the display unit 170 obtains display instructions from the controller 150, the display unit 170 displays, on a display screen, for example, information on fluorescence intensity, information on an electrocardiographic signal, time information, and the like, based on display information in the display instructions.

The operating unit 180 receives instructions, which are input through operations by a practitioner, and outputs the received instructions to the controller 150. The instructions include, for example, instructions to turn on/off the excitation light output from the light source 110, to change intensity, and the like. As intensity of the excitation light, it is possible to select at least one of two levels of intensity including a first intensity, which has a low power (for example, optical output of 1 mW or less) and is minimally-invasive with respect to a tissue and blood, and a second intensity, which has a high power and is approximately 1,000 times higher than the first intensity. The first intensity is selected when monitoring the pharmaceutical concentration and the contact state of the laser catheter 300 before therapy. The second intensity is selected when therapy is conducted. Note that the first intensity is a fixed value, and the second intensity may be variable.

### [Structure of laser catheter]

The the laser catheter 300 outputs an excitation light from the tip portion.
Fig. 4 is a sectional view showing the tip portion of the laser catheter.
The laser catheter 300 includes a catheter tube 310, a holder 320, an optical fiber 330, and an optical window 340.

The catheter tube 310 is a soft hollow tube, and is led to the inner wall of a cardiac-muscle tissue of the heart 10 of the patient 2. The catheter tube 310 has the optical fiber 330 therein.

The holder 320 is fixed to the catheter tube 310. The holder 320 holds the optical fiber 330 and the optical window 340 with respect to the catheter tube 310.

The optical fiber 330 is, for example, one quartz step index fiber having a core diameter of 133 µm and an outside diameter of 500 µm. The optical fiber 330 transmits the excitation light from the PDT apparatus 1. The optical fiber 330 outputs the transmitted excitation light, as an irradiation light 301, from the tip to the optical window 340. The beam diameter of the irradiation light 301 increases at the angle determined by the numerical aperture (NA) of the optical fiber 330. The tip of the optical fiber 330 is worked such that the beam diameter of the irradiation light 301 appropriately increases. The optical fiber 330 transmits the fluorescence, which is emitted from photo-sensitive pharmaceutical absorbed in a tissue and irradiated with an excitation light, to the PDT apparatus 1.

The optical window 340 is provided on the outermost of the tip portion of the laser catheter 300 such that the optical window 340 is optically connected to the tip of the optical fiber 330. The optical window 340 is made from a solid transparent material, for example, a glass material such as BK7. The optical window 340 as an irradiation section allows the irradiation light 301, which is output from the tip of the optical fiber 330, to pass through. The optical window 340 as a light-receiving section collects the fluorescence, which is emitted from the photo-sensitive pharmaceutical, on the tip of the optical fiber 330.

In order to detect fluorescence with a high SN (Signal-Noise) ratio, there is known a method of separately providing an irradiation fiber and a detection fiber in a laser catheter, and performing irradiation and light-reception, to thereby remove specular reflection light (see Japanese Patent Application Laid-open No. 2009-148550, paragraph [0037].).
Meanwhile, in the case of performing intracardiac therapy or diagnosis, in order to increase the curvature of a laser catheter, it is desirable that the diameter of a laser catheter be small. In the case of providing a plurality of optical fibers in a laser catheter, each optical fiber should be formed extra-finely, and thus a light having a necessary intensity may not be transmitted.
In view of the above, in diseases requiring intracardiac approaches such as, specifically, atrial fibrillation and ventricular flutter, it is desired that one optical fiber be in a laser catheter. Further, because it is necessary to detect fluorescence at intensity with a low power so as not to affect a living body, it is necessary to form a measurement system with a high SN (Signal-Noise) ratio by using one optical fiber.

In view of the above, according to the PDT apparatus 1 of this embodiment, the PBS 123 and the long pass filter 124 removes a specular reflection light on the fiber entrance edge, and the short pass filter 121 further removes a long-wavelength-side radiation component of a excitation light. With this structure, in the laser catheter 300, while the one optical fiber 330 doubles an irradiation fiber and a detection fiber, the detection unit 130 can detect fluorescence with a high SN ratio. As a result, it is possible to detect fluorescence with a low power so as not to affect a living body. Therefore, in the therapy and diagnosis of circulatory diseases, it is possible to perform minimally-invasive diagnoses with an extra-fine laser catheter with an increased curvature.

### [Operations of PDT apparatus]

Next, operations of the PDT apparatus 1 configured as described above will be described.

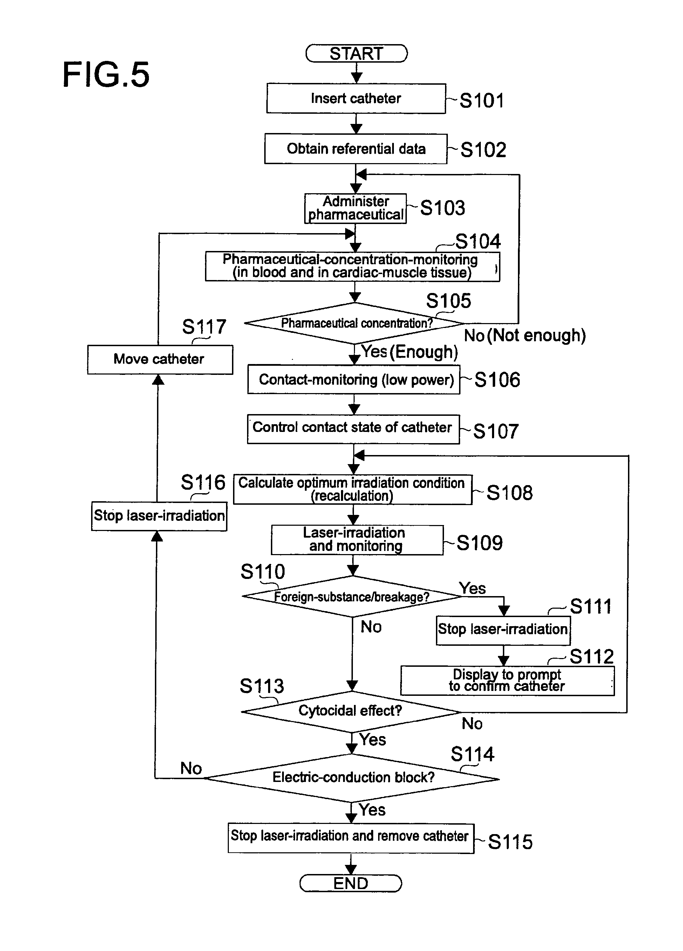

Fig. 5 is a flowchart showing operations of the PDT apparatus.

The operations of the PDT apparatus 1 will be described in the following order of (1) to (6).
(1) Preparation for PDT (Step S101 to Step S103)
(2) Pharmaceutical-concentration-monitoring operation (Step S104 to Step S105)
   In the pharmaceutical-concentration-monitoring operation, the light source 110 outputs an excitation light with a first intensity, and the controller 150 constantly calculates the pharmaceutical concentration based on fluorescence intensity detected by the detection unit 130, and determines whether to additionally administer pharmaceutical or not based on the calculated pharmaceutical concentration.
(3) Contact-monitoring operation (Step S106 to Step S108)
   In the contact-monitoring operation, the light source 110 outputs the excitation light with the first intensity, and the controller 150 determines the contact state of the laser catheter 300 with respect to a tissue inner wall based on fluorescence intensity detected by the detection unit 130, and calculates excitation-light-irradiation protocols (intensity, time, and the like).
(4) Foreign-substance/breakage-monitoring operation (Step S109 to Step S112)
   In the foreign-substance/breakage-monitoring operation, the light source 110 outputs the excitation light with a second intensity, and the controller 150 determines whether a foreign substance adheres to the tip of the laser catheter 300 for some reason or not and further determines whether a breakage occurs in the vicinity of the tip of the laser catheter 300 or not, during laser-irradiation at appropriate therapy protocols, based on the fluorescence intensity detected by the detection unit 130.
(5) Cytocidal-effect-determining operation (Step S113)
   In the cytocidal-effect-determining operation, the light source 110 outputs the excitation light with the second intensity, and the controller 150 determines whether there is a cytocidal effect on a tissue, on which the excitation light is being irradiated, or not based on the fluorescence intensity detected by the detection unit 130.
(6) Electric-conduction-block-formation determining operation (Step S114 to Step S117)
   An electric-conduction block is, as described above, a block in which cardiac-muscle tissues surrounding a hyperexcited site are necrotized, and in which conduction of electrical pulses from the hyperexcited site to the left atrium is blocked. Here, it is determined whether an electric-conduction block is formed or not by calculating, by the controller, temporal-change data of fluorescence intensity used in the cytocidal-effect-determining operation (Step S113), and electrocardiographic-wave data. In some cases, the laser catheter may be relocated in the electric-conduction block, the intensity of the light source 110 may be changed to the first intensity, and the similar process may be performed, to thereby determine whether an electric-conduction block is formed or not.

### [(1) Preparation for PDT]

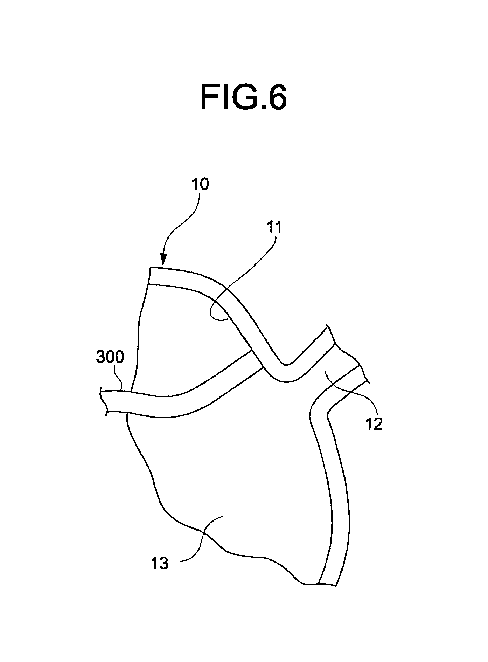

Fig. 6 is a schematic diagram showing a laser catheter inserted in a left atrium.
First, a practitioner such as a doctor inserts the laser catheter 300 in the heart 10 via a femoral vein or a jugular vein of the patient 2. The tip portion of the laser catheter 300 is disposed in the vicinity of a pulmonary vein 12 of an inner wall of a cardiac-muscle tissue 11 of the left atrium 13 (Step S101).

Subsequently, with reference to various referential data (Step S102), the practitioner administers photo-sensitive pharmaceutical to the patient 2 (Step S103). Here, the case where a dose of photo-sensitive pharmaceutical necessary for therapy is administered to the patient 2 at one time by intravenous injection will be described. The administered photo-sensitive pharmaceutical is diffused in blood and absorbed in a tissue.

### [(2) Pharmaceutical-concentration-monitoring operation]

Subsequently, the pharmaceutical-concentration-monitoring operation is performed.
First, the practitioner operates the operating unit 180, and inputs an excitation-light-output instruction with the low-power first intensity to the controller 150. The controller 150 obtains the excitation-light-output instruction, and then outputs the excitation-light-output instruction with the first intensity, to the light source 110. The light source 110 obtains the excitation-light-output instruction from the controller 150, and then outputs the excitation light with the first intensity. Tissues and blood are irradiated with the excitation light output from the light source 110 via the optical system 120 and the laser catheter 300. The photo-sensitive pharmaceutical, which is absorbed in a tissue and blood, absorbs the excitation light from the laser catheter 300, and emits fluorescence. The optical system 120 extracts the fluorescence emitted from the photo-sensitive pharmaceutical via the laser catheter 300, and the fluorescence enters the detection unit 130. The detection unit 130 detects the entered fluorescence, and outputs the detected fluorescence intensity to the controller 150 as an electrical signal.

The controller 150 calculates the fluorescence intensity based on the electrical signal obtained from the detection unit 130. The controller 150 starts to record, in the storage 160, the temporal change of the fluorescence intensity as a log in which the calculated fluorescence intensity is in relation with time information obtained from a timing measurement unit (not shown.). The controller 150 creates display information of the temporal change of the fluorescence intensity based on the calculated fluorescence intensity and elapsed time after a criterion time such as an intravenous-injection start time, and outputs a display instruction including the created display information to the display unit 170. The display unit 170 obtains the display instruction from the controller 150, and then displays the temporal change of the fluorescence intensity on a display screen based on the display information included in the display instruction. For example, the display unit 170 displays the temporal change of the fluorescence intensity on the display screen in a graph form.

Here, an example of the graph showing the temporal change of the fluorescence intensity will be described.

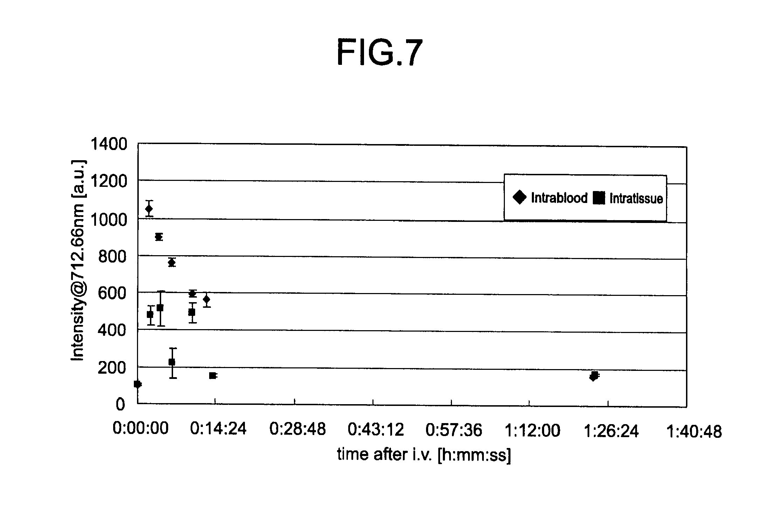

Fig. 7 is a graph showing a temporal change of fluorescence intensity.
Fig. 7 shows the temporal change of fluorescence intensity in the case where photo-sensitive pharmaceutical (Laserphyrin) is administered to a pig by intravenous injection (i.v.), and irradiation is performed with an excitation light, which is the same as the Q-band absorption spectrum of the pharmaceutical (semiconductor laser, emission wavelength with, for example 600 to 800 nm, preferably 660 to 680 nm, or more preferably 664 plus or minus 2, 400 µW). The tip portion of the laser catheter 300 is disposed in the right atrium of the pig.
The fluorescence intensity in blood monotonically decreases after the pharmaceutical administration. Meanwhile, the fluorescence intensity in a cardiac-muscle tissue increases for a predetermined time period after the pharmaceutical administration, and then decreases. Further, the fluorescence intensity in the blood is higher than the fluorescence intensity in the cardiac-muscle tissue.

Here, the relation between fluorescence intensity and pharmaceutical concentration will be described.

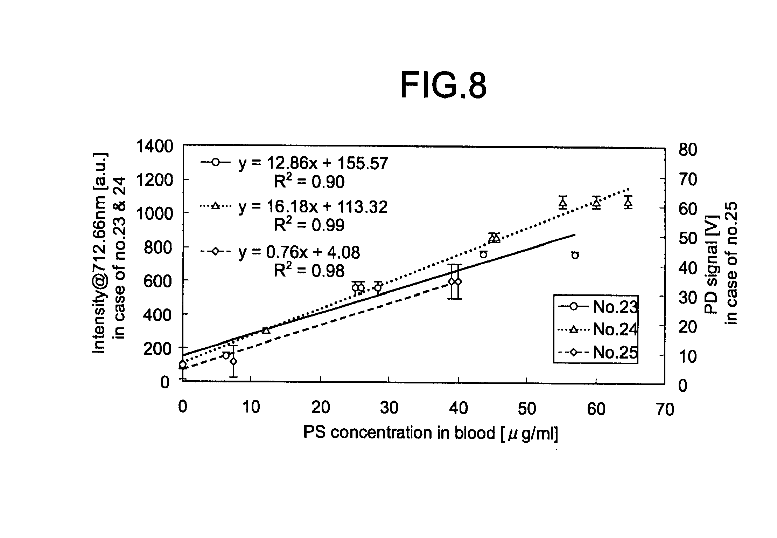

Fig. 8 is a graph showing a correlation between fluorescence intensity and pharmaceutical concentration.
Fig. 8 shows a correlation between absolute value of pharmaceutical concentration (PS concentration) obtained by a blood collection method, and the fluorescence intensity in the case where blood is irradiated with the excitation light as shown in Fig. 7. The absolute value of pharmaceutical concentration is almost the same as the fluorescence intensity. That is, it is possible to monitor pharmaceutical concentration in real time based on the constantly-calculated fluorescence intensity.

The controller 150 calculates pharmaceutical concentration in a tissue and in blood based on calculated fluorescence intensity (Step S104). The controller 150 starts to record, in the storage 160, the temporal change of the pharmaceutical concentration as a log in which the calculated pharmaceutical concentration is in relation with time information obtained from a timing measurement unit (not shown.). Further, the controller 150 creates display information of the temporal change of the pharmaceutical concentration based on the calculated pharmaceutical concentration and elapsed time after a criterion time such as an intravenous-injection start time, and outputs a display instruction including the created display information to the display unit 170. The display unit 170 obtains the display instruction from the controller 150, and then displays the temporal change of the pharmaceutical concentration on a display screen based on the display information included in the display instruction. For example, the display unit 170 displays the temporal change of the pharmaceutical concentration on the display screen in a graph form.

Here, an example of a graph showing the temporal change of the pharmaceutical concentration will be described.

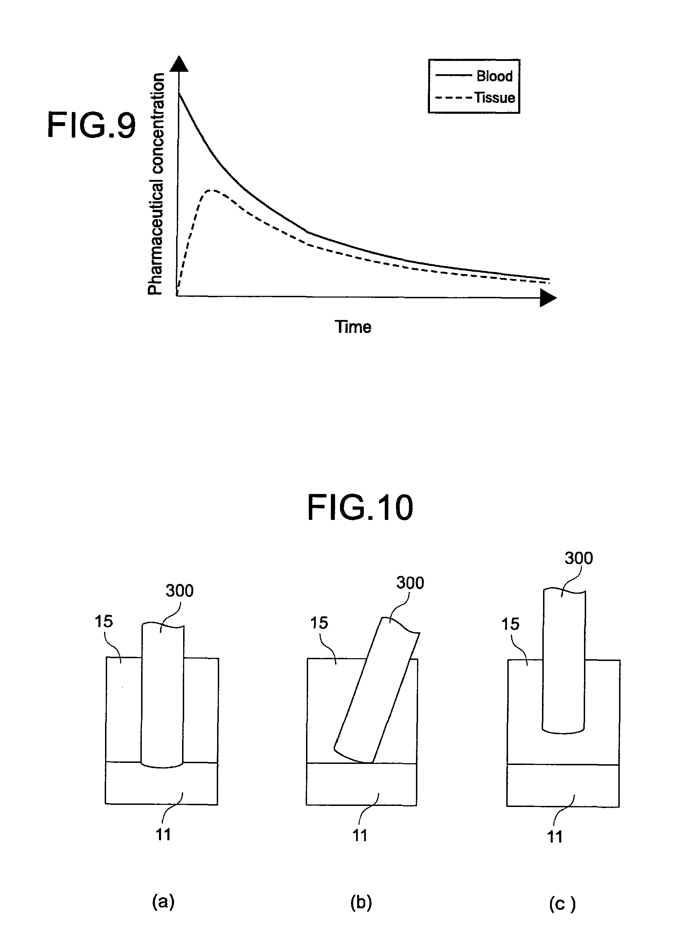

Fig. 9 is a graph showing a temporal change of pharmaceutical concentration.
As described above, the fluorescence intensity in blood is higher than the fluorescence intensity in a cardiac-muscle tissue, and, in addition, the fluorescence intensity correlates with the pharmaceutical concentration. Therefore, similar to the temporal change of the fluorescence intensity in blood, the pharmaceutical concentration in blood monotonically decreases after the pharmaceutical administration. Meanwhile, similar to the temporal change of the fluorescence intensity in a tissue, the pharmaceutical concentration in a tissue increases for a predetermined time period after the pharmaceutical administration, and then decreases. Further, the pharmaceutical concentration in blood is higher in level than the pharmaceutical concentration in a tissue.

The controller 150 determines whether the calculated pharmaceutical concentration is equal to or more than a threshold (Step S105). If the controller 150 determines that the pharmaceutical concentration is equal to or more than the threshold, the controller 150 estimates that the pharmaceutical concentration reaches a necessary value, and moves to the contact-monitoring operation (Step S105, Yes). Meanwhile, if the controller 150 determines that the pharmaceutical concentration is less than the threshold, the controller 150 estimates that the pharmaceutical concentration fails to reach the necessary value, creates display information for prompting to additionally administer the pharmaceutical, and outputs the display instruction including the created display information to the display unit 170. The display unit 170 obtains the display instruction from the controller 150, and then displays, based on the display information including the display instruction, information prompting the practitioner to additionally administer the photo-sensitive pharmaceutical (Step S105, No).

Note that, because the fluorescence intensity correlates with the pharmaceutical concentration, if the display unit 170 displays the fluorescence intensity on the display screen, a practitioner such as a doctor may estimate the pharmaceutical concentration based on the fluorescence intensity, even if the controller 150 does not calculate the pharmaceutical concentration.

Meanwhile, in general, as a method of monitoring the pharmaceutical concentration change in blood, there is known a method in which absorbance of blood, which is collected at regular time intervals after pharmaceutical administration, is measured. However, in this method, the plot number is limited because the collectable blood volume is limited, and in addition, it is not possible to measure the concentration in real time.
Alternatively, there is known a method in which a bypass pathway is prepared outside of a body, blood passing through the pathway is irradiated with light, and fluorescence intensity is observed, to thereby monitor the pharmaceutical concentration change. However, it is necessary to pay attention to hygiene in this method.
Further, as a method of monitoring pharmaceutical concentration in a tissue, there is known a method in which part of carbon in pharmaceutical is transformed into isotope, the isotope is simultaneously administered, and pharmaceutical concentration in each tissue is monitored based on a radiation quantity (CANCER RESEARCH 50. 3985-3990, July 1, 1990, Tissue Distribution and Photosensitizing Properties of Mono-L-aspartyl Chlorin e6 in a Mouse Tumor Model, Charles J. Corner and Angela Ferrario). However, this method involves radiation exposure problems, and involves a problem in that only a concentration may be monitored macroscopically.

To the contrary, according to the pharmaceutical-concentration-monitoring operation of this embodiment, by calculating the temporal change of fluorescence intensity, the temporal change of pharmaceutical concentration, which correlates with fluorescence intensity, may be calculated. Therefore the pharmaceutical concentration in a tissue and blood may be monitored in real time. Further, the pharmaceutical-concentration-monitoring operation of this embodiment is less invasive than the conventional monitoring method, and is capable of monitoring temporal changes of pharmaceutical concentration stably and reproducibly. Further, because the temporal change of pharmaceutical concentration is monitored via a catheter by using the excitation light from the light source 110 of the PDT apparatus 1, it is not necessary to additionally provide a pharmaceutical concentration detecting apparatus, to thereby enable a low-cost and space-saving apparatus. Further, because the pharmaceutical concentration may be monitored in real time, determination of additional pharmaceutical administration may be assisted in real time.

Further, the pharmaceutical-concentration-monitoring operation of this embodiment may be performed not only in PDT but also in therapy or diagnosis using a pharmaceutical, which absorbs an excitation light and emits fluorescence. In therapy or diagnosis using a pharmaceutical, it is important to grasp a pharmaceutical dynamic state (pharmaceutical delivery). According to the pharmaceutical-concentration-monitoring operation of this embodiment, pharmaceutical concentration in an intended tissue may be measured microscopically via a catheter in real time, and dynamic states of various pharmaceuticals may be grasped. Further, because minimally-invasive monitoring is enabled, the pharmaceutical-concentration-monitoring operation of this embodiment has a great advantage and is suitable for practical use. Further, the pharmaceutical-concentration-monitoring operation of this embodiment may be performed in a system (DDS, Drug Delivery System) in which pharmaceutical is delivered to only a certain location, and is useful to estimate whether pharmaceutical reaches actually and locally.

### [(3) Contact-monitoring operation]

Subsequently, the contact-monitoring operation is performed.

Figs. 10 are schematic diagrams showing contact states of the laser catheter.
The laser catheter 300 is preferably disposed such that the tip portion as a light-emitting portion contacts the inner wall of the cardiac-muscle tissue 11 upright (see Fig. 10(a), hereinafter referred to as "upright-contact state".). This state is preferable so as to remove intraatrial blood 15 from the tip portion of the laser catheter 300, and to prevent activation of photo-sensitive pharmaceutical in the intraatrial blood 15. Further, this state is preferable so as to selectively activate photo-sensitive pharmaceutical absorbed in a tissue when the tip portion of the laser catheter 300 directly contacts a tissue.
However, it is difficult to recognize the precise contact state of the tip portion of the laser catheter 300 radiographically or tactually. Because of this, actually, it is not always true that the tip portion of the laser catheter 300 is in the upright-contact state with respect to a tissue. The blood 15 may exist between the tip portion of the laser catheter 300 and a tissue, and the tip portion may be in the blood (see Fig. 10(c), hereinafter referred to as "non-contact state".). Alternatively, the tip portion of the laser catheter 300 may contact a tissue in a slanting direction, and the blood 15 may partially exist in a gap between the tip portion and the tissue (see Fig. 10(b), hereinafter referred to as "slanting-contact state".).
In the contact-monitoring operation, such contact states of the tip portion of the laser catheter 300, that is, the contact states and the non-contact state, are monitored, the contact angle (upright-contact state, slanting-contact state) in the case of the contact states is monitored, and the like. Note that, in this specification, the "contact angle" not only means a narrowly-defined angular value, but also means a widely-defined contact angle, in which the contact state of the tip portion of the laser catheter 300 with respect to a tissue is upright or slanting.

Continuously, the light source 110 outputs the excitation light with the first intensity to the optical system 120, the controller 150 calculates fluorescence intensity and pharmaceutical concentration, and the display unit 170 displays the temporal change of fluorescence intensity on the display screen. For example, the display unit 170 displays the temporal change of fluorescence intensity on the display screen as a graph.

Here, an example of a graph showing the temporal change of fluorescence intensity will be described.

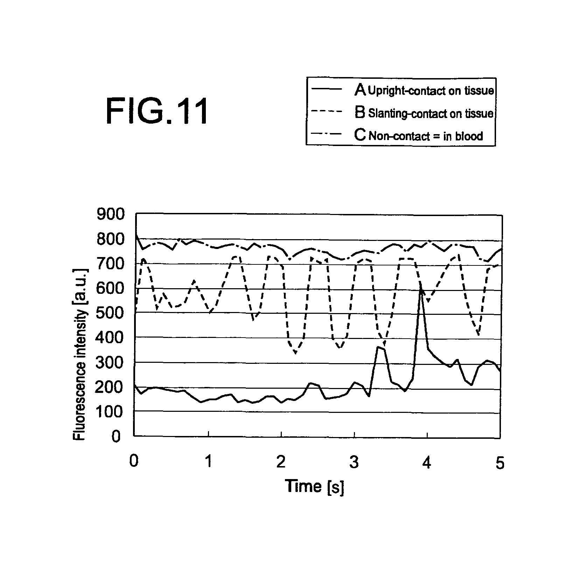

Fig. 11 is a graph showing the temporal change of fluorescence intensity.
Fig. 11 is a graph showing the temporal change of fluorescence intensity under the condition same as Fig. 7. In the graph, the line A shows low fluorescence intensity, the line C shows high fluorescence intensity, and the line B fluctuates between the fluorescence intensity of the line A and the fluorescence intensity of the line C.
Note that, in Fig. 11, in order to make the description clear, the temporal change of fluorescence intensity in the case where the tip portion of the laser catheter 300 is in the upright-contact state, the temporal change of fluorescence intensity in the case where the tip portion of the laser catheter 300 is in the slanting-contact state, and the temporal change of fluorescence intensity in the case where the tip portion of the laser catheter 300 is in the non-contact state are shown in one graph. However, actually, one of them is displayed according to the contact state of the tip portion of the laser catheter 300.
The line A will be reviewed. Here, as shown in Fig. 7, the fluorescence intensity in a tissue is smaller than the fluorescence intensity in blood. Therefore, it is thought that the line A shows the fluorescence intensity in the case where the laser catheter 300 irradiates a tissue with the excitation light. So, in the case where the fluorescence intensity of the line A is calculated, it is thought that the fluorescence intensity in a tissue is reflected in the result because the tip portion of the laser catheter 300 is in the upright-contact state with respect to a tissue.
The line C will be reviewed. Here, as shown in Fig. 7, the fluorescence intensity in blood is larger than the fluorescence intensity in a tissue. Therefore, it is thought that the line C shows the fluorescence intensity in the case where the laser catheter 300 irradiates blood with the excitation light. So, in the case where the fluorescence intensity of the line C is calculated, it is thought that the fluorescence intensity in blood is reflected in the result because the tip portion of the laser catheter 300 is in the non-contact state with respect to a tissue.
The line B will be reviewed. Because the line B is between the fluorescence intensity of the line A and the fluorescence intensity of the line C, it is thought that the tip portion of the laser catheter 300 is in the slanting-contact state with respect to a tissue. Further, because a contact-target object of the tip portion of the laser catheter 300 is a moving cardiac-muscle tissue, the laser catheter 300 follows the movement of the tissue to thereby move. As a result, in the case where the tip portion of the laser catheter 300 contacts a tissue in a slanting manner, it is likely that the blood volume between the tip portion of the laser catheter 300 and a tissue changes during measurement. In addition, the blood-flow volume in a cardiac-muscle tissue changes and the intraatrial blood-flow volume changes because of heartbeat. Affected by them, the fluctuation of the fluorescence intensity of the line B is larger than the line A and the line C.
Further, in the case where the tip portion of the laser catheter 300 contacts a tissue in any state (upright-contact state, slanting-contact state), the laser catheter 300 may be affected by the movement of the cardiac-muscle tissue. That is, the contact state of the tip portion of the laser catheter 300 fluctuates between the contact states (upright-contact state, slanting-contact state) and the non-contact state. In this case, the fluorescence intensity fluctuates largely. Therefore, it is determined whether the laser catheter 300 follows the movement of a cardiac-muscle tissue or not based on fluctuation of the fluorescence intensity shown in a waveform. For example, in the line A of the graph, the high fluorescence intensity after four seconds after pharmaceutical administration and in the vicinity thereof shows that the tip portion of the laser catheter 300 momentarily moves from the upright-contact state to the non-contact state and returns to the upright-contact state again.

Based on the calculated fluorescence intensity, the controller 150 determines the contact state of the tip portion of the laser catheter 300 (contact/non-contact states, contact angle in case of contact state) (Step S106).
Specifically, in the case where the controller 150 determines that the calculated fluorescence intensity is equal to or larger than a first threshold, the controller 150 determines the non-contact state (line C). In the case where the controller 150 determines that the minimum value of the fluorescence intensity is equal to or smaller than a second threshold, which is smaller than the first threshold, the controller 150 determines the upright-contact state (line A). In the case where the controller 150 determines that the fluorescence intensity periodically fluctuates between the first threshold and the second threshold, the controller 150 determines the slanting-contact state (line B).
The controller 150 informs the practitioner the determined contact state by using the display unit 170. Specifically, when the controller 150 determines the slanting-contact state or the non-contact state, the controller 150 creates display information for prompting to change the contact state of the tip portion of the laser catheter 300, and outputs a display instruction including the created display information to the display unit 170. The display unit 170 obtains the display instruction from the controller 150, and then displays information for prompting a practitioner to change the contact state of the tip portion of the laser catheter 300 based on the display information in the display instruction (Step S107). The practitioner operates a handpiece or the like (not shown.) provided on the laser catheter 300, to thereby change the contact state of the tip portion of the laser catheter 300 with respect to a tissue.

The controller 150 continuously calculates fluorescence intensity and pharmaceutical concentration. The controller 150 refers to fluorescence intensity and pharmaceutical concentration stored in the storage 160. The controller 150 calculates the blood volume in the gap between the tip portion of the laser catheter 300 and a tissue based on the referred fluorescence intensity. The controller 150 calculates excitation-light-irradiation protocols during the therapy, that is, the second intensity of the excitation light, the irradiation time, and the like, based on the calculated blood volume and the referred pharmaceutical concentration (Step S108).
For example, in the case where the tip portion of the laser catheter 300 is in the slanting-contact state or the non-contact state and where blood exists in the gap, the loss of the excitation light (excitation light which does not reach tissue) is considered based on the blood volume, and the excitation-light-irradiation protocols are set, in which the second intensity is high and in which the irradiation time is long. The controller 150 calculates the excitation-light-irradiation protocols, creates display information on the irradiation protocols, and outputs display instruction including the created display information to the display unit 170. The display unit 170 obtains the display instruction from the controller 150, and then displays information on the excitation-light-irradiation protocols (second intensity, irradiation time) based on the display information in the display instruction.
As described above, the controller 150 calculates the pharmaceutical concentration and the blood volume based on the fluorescence intensity, and calculates the excitation-light-irradiation protocols based on the calculated pharmaceutical concentration and blood volume. That is, the controller 150 is capable of calculating the excitation-light-irradiation protocols based on the fluorescence intensity.

Note that, because the temporal change of fluorescence intensity differs depending on the contact state of the tip portion of the laser catheter 300, if the temporal change of fluorescence intensity is displayed on the display screen by the display unit 170, it is possible for a practitioner to estimate a contact state based on the temporal change of fluorescence intensity even if the controller 150 does not determine the contact state.

Meanwhile, in the field of circulatory disease, it is important to determine, in real time, the contact state of the tip portion of a catheter with respect to the intended tissue, the blood volume in a gap, and presence/absence of a foreign-substance/breakage in order to ensure safety and reliability. Further, in the case where the intended tissue is a movable target such as a cardiac-muscle tissue, it is necessary to determine the contact state in detail, in which a laser catheter follows the movement of the tissue, to reliably perform therapy. In the past, it is known to determine the contact state of a catheter by, for example, securing a transparent zone by removing blood, radioscopy, potential measurement (impedance measurement), potential mapping, temperature measurement, dynamic measurement (pressure, stress), reflected light measurement using a polychromatic light source, and the like. However, in the field of therapy and diagnosis via a catheter, it is difficult to determine the tip state of the catheter in blood, and a technique capable of determining the contact state in detail has not been developed yet. Each of the above-mentioned conventional methods is capable of determining the contact state roughly, and, in addition, has many problems as follows.
Securing a transparent zone by removing blood is a method in which a blood flow is temporarily blocked by using a balloon, saline or the like is flowed from a catheter tip portion to thereby secure a transparent zone, and a contact state is observed by using an angioscope. However, this method may lead to a peripheral-vessel-ischemia state.
With radioscopy, because of lacking accuracy, it is difficult to determine the distance of a gap between a catheter and a tissue, and the blood volume in the gap between the catheter and the intended tissue. Further, in the case of a moving tissue, it is not clear that the tip of a catheter follows the movement. As a result, the tip of a catheter may break blood (in case of intracardiac therapy) or blood-vessel wall (in case of intravascular therapy). Further, the amount of energy input in an intended tissue decreases below an estimated amount, and an enough therapeutic effect may not be achieved. Further, the biggest problem is that only a doctor, who has a knowledge of anatomy and is well-experienced (tactile impression when touching), can make a determination, subjectively (see Japanese Patent Application Laid-open No. 2007-525263).
Potential measurement (impedance measurement) is a method in which, since a cardiac-muscle tissue contracts and moves because of potential propagation, the contact state with respect to a cardiac-muscle tissue is determined by measuring the potential. However, in the case of performing an optical therapy, the tip portion of a catheter (contact portion with respect to cardiac-muscle tissue) is an optical window. Because of this, a potential-measured site may be provided on a portion other than the tip portion of the catheter. As a result, a light-irradiated site does not coincide with a potential-measured site, a diagnosis-target zone does not coincide with a therapy-target zone, and the therapy may not be performed precisely. Further, an electrode area is made smaller, and angle determination accuracy may thus be decreased. Further, because electric measurement is performed, there may be an effect of electromagnetic interference (see Japanese Patent Application Laid-open No. 2008-531170).
Potential mapping is a method in which potential measurement is three-dimensionally developed. However, a conventional apparatus lacks a resolution of determining a contact state in detail. Further, it takes time to perform determination, and an anthropogenic influence may occur because of an excessive contact pressure (see Japanese Patent Application Laid-open No. 2008-531170). Further, if a potential measuring catheter is displaced, a mapping image may not coincide with an actual position. Further, because electric measurement is performed, there may be an effect of electromagnetic interference (see Japanese Patent Application Laid-open No. 2008-531170).
Temperature measurement is a method in which, with respect to diseases including a vascular occlusion, an occlusion is determined by measuring temperature (see Japanese Patent Application Laid-open No. 2007-525263). However, this is a diagnostic method for only occlusions, and is not applicable to diseases including no occlusion zone such as, for example, atrial fibrillation and ventricular flutter. Further, unnecessary heat may be provided on a normal blood-vessel wall.
Dynamic measurement (pressure, stress) is a method in which a pressure sensor or a stress sensor is mounted on a catheter, and a contact-target object is determined (see Japanese Patent Application Laid-open No. 2009-542371, US Patent No. 6696808, US Patent Application Laid-open No. 2008/0009750, WIPO Publication No. 01/33165) However, the tip portion of the catheter may be larger, and there may be an effect of electromagnetic interference (see Japanese Patent Application Laid-open No. 2008-531170).
Reflected light measurement by using a polychromatic light source is a method in which absorption coefficients different from wavelengths are used. Specifically, by using a polychromatic light source, a tissue is determined based on reflection ratio differences of the respective wavelengths (see Japanese Patent No. 4261101). According to this method, although the blood volume between a catheter and a tissue may be estimated, an optical system may be complicated, an apparatus may be made larger, and the cost may be increased because a plurality of light sources are provided.

To the contrary, according to the contact-monitoring operation of this embodiment, by detecting fluorescence intensity, it is possible to determine the contact state with respect to an intended tissue and following-movements via a catheter in real time. This method is minimally invasive because it is not necessary to remove blood and the like. Further, because excitation-light-irradiation protocols may be calculated based on the determined contact state and the like, therapy and diagnosis may be assisted safely and reliably.

### [(4) Foreign-substance/breakage-monitoring operation]

During the photodynamic therapy, the foreign-substance/breakage-monitoring operation is performed.
First, a practitioner refers to excitation-light-irradiation protocols displayed on the display unit 170, and operates the operating unit 180 to thereby input an excitation-light-output instruction with the high-power second intensity in the controller 150. The controller 150 obtains the excitation-light-output instruction, and then outputs the excitation-light-output instruction with the second intensity to the light source 110. The light source 110 obtains the excitation-light-output instruction from the controller 150, and then outputs the excitation light with the second intensity. A tissue is irradiated with the excitation light output from the light source 110 via the optical system 120 and the laser catheter 300, and photodynamic therapy is performed (Step S109).

Based on an electrical signal obtained from the detection unit 130, the controller 150 calculates fluorescence intensity. The controller 150 creates display information of the temporal change of the fluorescence intensity based on the calculated fluorescence intensity and elapsed time after a criterion time such as an intravenous-injection start time, and outputs a display instruction including the created display information to the display unit 170. The display unit 170 obtains the display instruction from the controller 150, and then displays the temporal change of the fluorescence intensity on a display screen based on the display information included in the display instruction.

The controller 150 determines whether the calculated fluorescence intensity is equal to or more than a threshold (Step S110). The threshold is, for example, a value equal to or more than the multiple of the normal fluorescence intensity.

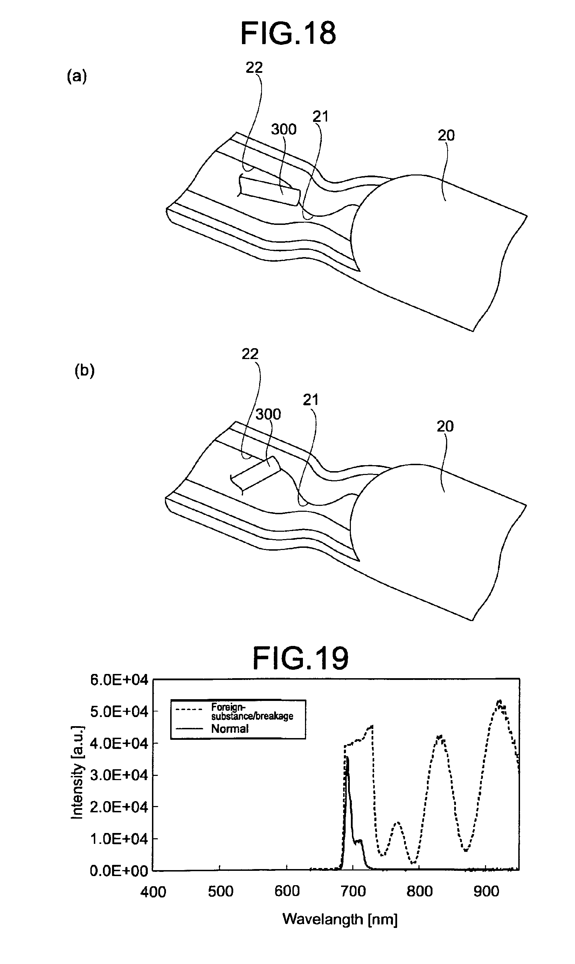

Fig. 19 is a graph showing the relation between wavelength and fluorescence intensity.
Fig. 19 shows the relation between wavelength and fluorescence intensity of a laser catheter, which may contact a foreign substance or may be broken, and those of a normal laser catheter. It is understood that, in the case where the tip portion of a laser catheter contacts a foreign substance other than a living body tissue or is broken, the fluorescence intensity thereof is larger than the normal fluorescence intensity.

The controller 150 determines that the fluorescence intensity is equal to or more than the threshold, that is, determines that the fluorescence intensity is increased to equal to or more than the multiple of the previous fluorescence intensity such that the current fluorescence intensity ignores the previous fluorescence intensity, and then the controller 150 estimates that there is a foreign substance or a breakage (Step S110, Yes). If the controller 150 estimates that there is a foreign substance or a breakage, the controller 150 creates display information on generation of a foreign-substance/breakage to stop the excitation light irradiation, and outputs display instruction including the created display information to the display unit 170. The display information on generation of a foreign-substance/breakage includes information to stop the excitation light irradiation, to reset irradiation time, to reset an irradiation power, to prompt to check the laser catheter 300, and the like. When the display unit 170 obtains the display instruction from the controller 150, based on the display information in the display instruction, the display unit 170 displays information to stop the excitation light irradiation (Step S111) and information on generation of a foreign-substance/breakage (Step S112) to a practitioner.
Note that, in the case where the controller 150 detects abnormal intensity increase of an arbitrary wavelength other than the fluorescence wavelength (excitation light wavelength or the like), the controller 150 estimates that there is a foreign substance or a breakage (Step S110, Yes), and may perform the similar processing (Step S111, Step S112).

Meanwhile, in the case where the controller 150 does not determine that the fluorescence intensity is equal to or more than the threshold within a predetermined time period, the controller 150 estimates that there is no foreign substance or breakage, and moves to the cytocidal-effect-determining operation (Step S110, No).

Note that, because generation of a foreign substance or a breakage is estimated when the fluorescence intensity exceeds a predetermined threshold, if the display unit 170 displays the fluorescence intensity on the display screen, a practitioner may estimate generation of a foreign substance or a breakage based on the fluorescence intensity, even if the controller 150 does not estimate generation of a foreign substance or a breakage.

Further, in the case where a plurality of catheters are disposed in a cardiac cavity in addition to the laser catheter 300, the laser catheter 300 may contact another catheter. For example, if the laser catheter 300 emits a light in the state where the laser catheter 300 contacts another catheter disposed in a cardiac cavity, both of the catheters may lose their functions. If a practitioner is keep on emitting an excitation light without noticing the abnormal situation of the tip portion of the laser catheter 300, the tip portion of the laser catheter 300 may generate heat to thereby be in danger of thermally damaging a living body. Further, a catheter being contacted may lose its function.

According to the foreign-substance/breakage-monitoring operation of this embodiment, because a strong reflected light is measured when the catheter contacts any object other than a living body tissue, it is possible to estimate generation of a foreign-substance/breakage via a catheter in real time. Because of this, it is possible to prompt a practitioner to check the laser catheter 300, and thus it is possible to perform the therapy very safely without causing danger to a patient.

### [(5) Cytocidal-effect-determining operation]

Subsequently, the cytocidal-effect-determining operation is performed.
In photodynamic therapy, photo-sensitive pharmaceutical absorbed in a tissue absorbs the excitation light from the laser catheter 300 to gain energy, and changes from the ground state to the singlet excited state. Most of the energy changes from the singlet excited state to the triplet excited state because of intersystem crossing, but the rest part returns from the singlet state to the ground state, and emits fluorescence at this time. Further, when photo-sensitive pharmaceutical in the triplet excited state clashes triplet oxygen, the photo-sensitive pharmaceutical transfers energy to oxygen, and creates strong oxidizer singlet oxygen. The oxidizer breaks a tissue, and, in addition, breaks photo-sensitive pharmaceutical (bleaching). If the bleaching occurs, the effective pharmaceutical amount is decreased, and thus the fluorescence amount is also decreased. Therefore, decrease of the fluorescence amount indicates bleaching and a tissue injury amount. The optical system 120 extracts the fluorescence emitted from the photo-sensitive pharmaceutical via the laser catheter 300, and the fluorescence enters the detection unit 130. The detection unit 130 detects the fluorescence entered from the optical system 120, and outputs the intensity of the detected fluorescence to the controller 150 as an electrical signal.

Continuously, the light source 110 outputs the excitation light with the second intensity to the optical system 120, the controller 150 calculates the fluorescence intensity, and the display unit 170 displays the temporal change of fluorescence intensity on the display screen. For example, the display unit 170 displays the temporal change of fluorescence intensity on the display screen as a graph.

Here, an example of a graph showing the temporal change of fluorescence intensity will be described.

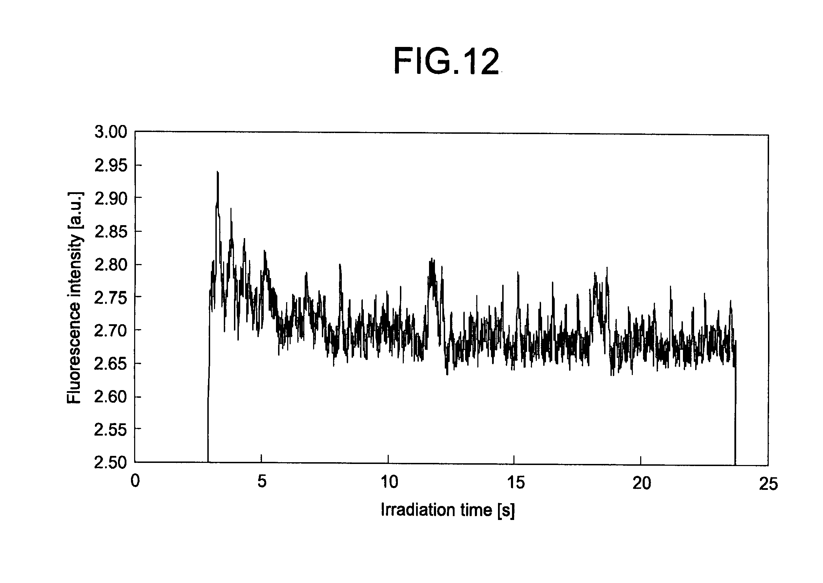

Fig. 12 is a graph showing the temporal change of fluorescence intensity.
Fig. 12 shows the temporal change of fluorescence intensity in the case where the excitation light is emitted for 20 seconds after 20 minutes pass after photo-sensitive pharmaceutical is administered in a pig by intravenous-injection. Since decrease of a fluorescence amount indicates bleaching and a tissue injury amount as described above, by displaying an attenuation curve of the fluorescence intensity, the PDT process may be displayed in real time.

The controller 150 determines whether the calculated fluorescence intensity is attenuated below a threshold (Step S113). If the controller 150 determines that the fluorescence intensity is attenuated below the threshold, the controller 150 estimates that there is a cytocidal effect on a tissue irradiated with the excitation light (Step S113, Yes). Then, the controller 150 creates display information on an index of a cytocidal effect, and outputs a display instruction including the created display information to the display unit 170. The display unit 170 obtains the display instruction from the controller 150, and then displays information on an index of a cytocidal effect for a practitioner based on the display information in the display instruction. The practitioner refers to the information on an index of a cytocidal effect displayed on the display unit 170, and moves to the electric-conduction-block-formation determining operation.

Meanwhile, if the controller 150 does not determine that the fluorescence intensity is decreased below the threshold within a predetermined time period, the controller 150 creates display information to prompt to extend the excitation light irradiation and to reset the light intensity based on the calculated fluorescence intensity, and outputs display instruction including the created display information to the display unit 170 (Step S113, No). When the display unit 170 obtains the display instruction from the controller 150, the display unit 170 displays information to prompt a practitioner to extend the excitation light irradiation and to reset the light intensity based on the display information in the display instruction. After a predetermined time period passes after outputting the display instruction, the controller 150 moves to the operation of Step S108.

Note that, because generation of a cytocidal effect is estimated when the fluorescence intensity is attenuated below a predetermined threshold, if the display unit 170 displays the fluorescence intensity on the display screen, a practitioner may estimate whether there is a cytocidal effect or not based on the fluorescence intensity, even if the controller 150 does not estimate whether there is a cytocidal effect or not.

According to the cytocidal-effect-determining operation of this embodiment, based on fluorescence intensity correlated with pharmaceutical concentration, it is possible to measure injury in a cardiomyocyte, which progresses in a tissue irradiated with the excitation light, that is, to measure a therapeutic effect, via a catheter in real time, and thus the therapy is performed reliably.

### [(6) Electric-conduction-block-formation determining operation]

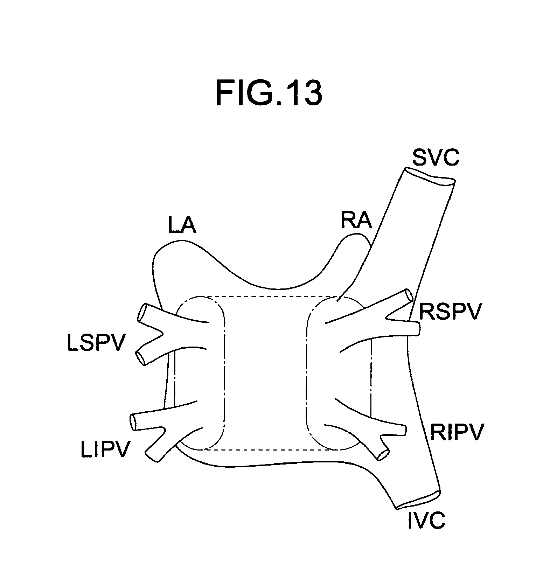

Subsequently, the electric-conduction-block-formation determining operation is performed.
In the electric-conduction-block-formation determining operation, the fluorescence time-waveform used in the cytocidal effect determination is in synchronization with electrocardiogram (ECG. ECG obtaining method will be described later.). The controller 150 analyzes the phase difference between the ECG R-wave and the fluorescence peak intensity in the interval between R-waves to thereby determine whether an electric-conduction block is formed. In some cases, the laser catheter 300 may be relocated in an electric-conduction block (in dashed-dotted line of Fig. 13), the excitation light output may be changed to the first intensity, and the fluorescence time-waveform, which is measured in low power, may be in synchronization with ECG to thereby perform analysis. The procedure in the case of relocating a laser catheter for measurement is as follows.

First, a practitioner disposes the tip portion of the laser catheter 300 in an electric-conduction block (in dashed-dotted line of Fig. 13) or on an excitation-light-irradiated site. Then, the practitioner operates the operating unit 180 to thereby input an excitation-light-output instruction with the low-power first intensity to the controller 150. The controller 150 obtains the excitation-light-output instruction, and then outputs the excitation-light-output instruction with the first intensity to the light source 110. The light source 110 obtains the excitation-light-output instruction from the controller 150, and then outputs the excitation light with the first intensity. A tissue is irradiated with the excitation light output from the light source 110 via the optical system 120 and the laser catheter 300. Photo-sensitive pharmaceutical, which is absorbed in a tissue, absorbs the excitation light from the laser catheter 300, and emits fluorescence. The optical system 120 extracts the fluorescence emitted from the photo-sensitive pharmaceutical via the laser catheter 300, and the fluorescence enters the detection unit 130. The detection unit 130 detects the fluorescence entered from the optical system 120, and outputs the detected fluorescence intensity to the controller 150 as an electrical signal. The controller 150 calculates fluorescence intensity based on the obtained electrical signal.

Meanwhile, the electrocardiograph 140 obtains the electrocardiographic signal, and supplies the obtained electrocardiographic signal to the controller 150. The controller 150 creates display information based on the calculated fluorescence intensity and the obtained electrocardiographic signal, and outputs display instruction including the created display information to the display unit 170. The display unit 170 obtains the display instruction from the controller 150, and displays the correlation between the fluorescence intensity and the electrocardiogram R-wave on the display screen based on the display information in the display instruction.

Here, the correlation between fluorescence intensity and ECG R-wave will be described.

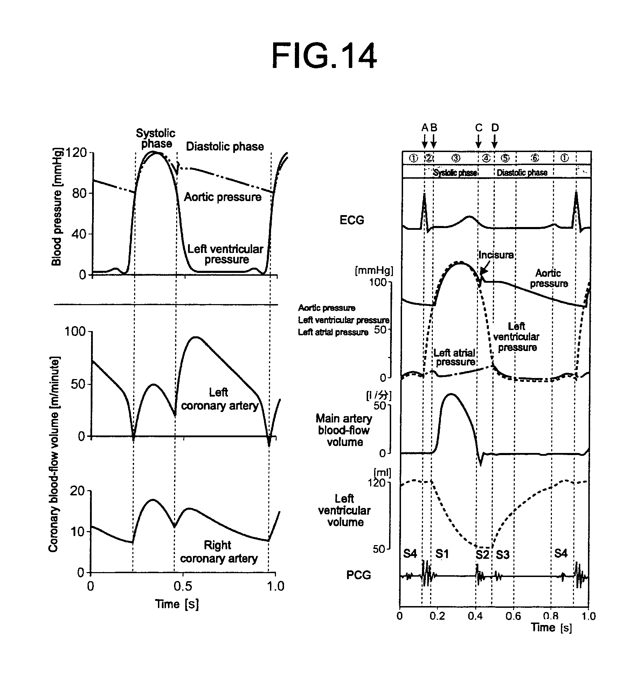

Fig. 14 is a diagram showing the relation of ECG, intracardiac pressure, and coronary blood-flow volume, which is dominant in the blood-flow volume in a cardiac-muscle tissue, and being a prerequisite knowledge described in "Essential Anatomy and Physiology (Essensharu Kaibo Seirigaku)" (Gakken Medical Shujunsha Co., Ltd., 2001), which is effective in the following description.
As shown in Fig. 14, the temporal change of the intracardiac blood-flow volume is different from the blood-flow volume in a cardiac-muscle tissue. While the intracardiac blood-flow volume has a peak at the time when it coincides with R-wave, the blood-flow volume in a right-sided cardiac-muscle tissue has a first peak at the time when about 200 ms pass after R-wave, and a second peak at the time when about 400 ms pass after R-wave.

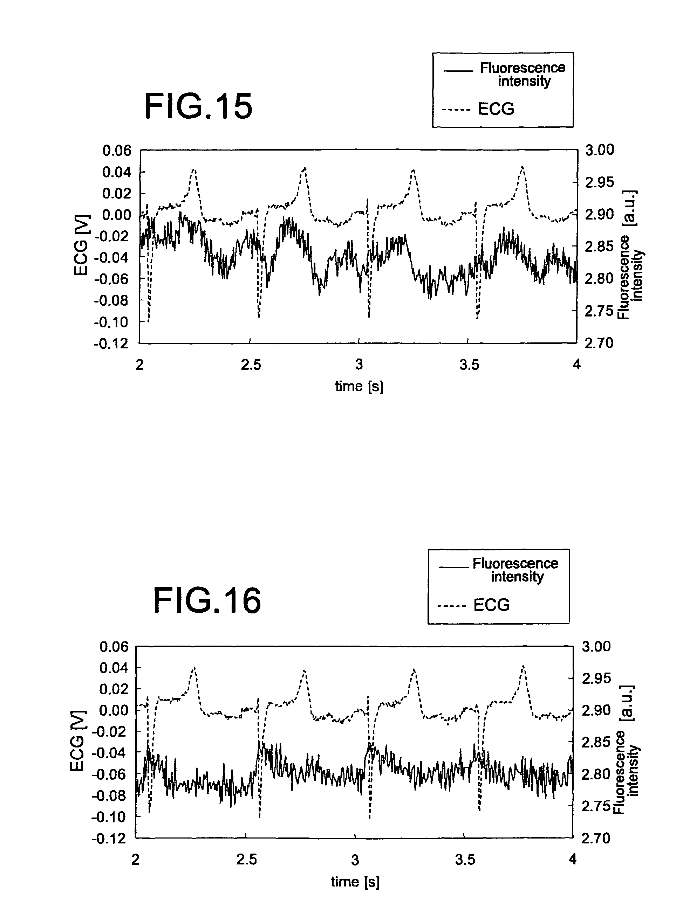

Fig. 15 is a diagram showing the correlation between fluorescence intensity and R-wave when the laser catheter is in the upright-contact state.
The correlation between fluorescence intensity (for example, irradiation power of 900 mW) and R-wave when the tip portion of the laser catheter 300 is in the upright-contact state will be described. In the upright-contact state, fluorescence peaks are observed after 100 ms pass after R-wave and after 400 ms pass after R-wave. Note that, in the case where the catheter is disposed left-sided, the fluorescence intensity changes in proportion to the left coronary blood-flow volume of Fig. 14. A ventricle contracts when R-wave appears, and blood is supplied to a whole body (including cardiac-muscle tissue). Since blood includes photo-sensitive pharmaceutical, the fluorescence intensity in a cardiac-muscle tissue is highest when blood is supplied to blood vessels of a cardiac muscle. As a result, the peak of fluorescence intensity appears for a predetermined time period after appearance of R-wave.

Fig. 16 is a diagram showing the correlation between fluorescence intensity and R-wave when the laser catheter is in the slanting-contact state.
The correlation between fluorescence intensity (for example, irradiation power of 900 mW) and R-wave when the tip portion of the laser catheter 300 is in the slanting-contact state will be described. In the slanting-contact state, because blood exists in a gap and the intracardiac blood-flow volume is dominant, the fluorescence intensity peak coincides with R-wave.
As described above, the phase difference between R-wave and fluorescence intensity peak in the upright-contact state is obviously different from the phase difference between R-wave and fluorescence intensity peak in the slanting-contact state, and the phase difference is constant if the contact state is maintained.

In view of this, the controller 150 determines whether the phase difference between fluorescence intensity and R-wave is constant based on the calculated fluorescence intensity and the obtained electrocardiographic signal, to thereby determine whether an electric-conduction block is formed or not (Step S114). If the controller 150 determines that the phase difference between fluorescence intensity and R-wave is constant, the controller 150 determines that an electric-conduction block is yet to be formed (Step S114, No), and causes the display unit 170 to display information to prompt a practitioner to stop the excitation light irradiation (Step S116) and to move the laser catheter 300 (Step S117).
The practitioner refers to the information displayed on the display unit 170, stops excitation light irradiation once, and moves the laser catheter 300. Then, the processing of Step S104 and thereafter are performed again.

Fig. 13 is a schematic diagram showing a movement track of the laser catheter.
A practitioner moves the tip portion of the laser catheter 300 so as to surround a hyperexcited site in pulmonary veins (PV) (dashed-dotted line or dashed line in Fig. 13).

Meanwhile, if the controller 150 determines that the phase difference between fluorescence intensity is R-wave is not constant, the controller 150 determines that an electric-conduction block is formed (Step S114, Yes), creates a display instruction to prompt a practitioner to stop excitation light irradiation and to remove the laser catheter 300, and outputs a display instruction including the created display information to the display unit 170. When the display unit 170 obtains the display instruction from the controller 150, the display unit 170 displays information to prompt a practitioner to stop excitation light irradiation and to remove the laser catheter 300 on the display screen based on the display information in the display instruction, and stops the processing (Step S115).

Here, the principle, in which the controller 150 determines that an electric-conduction block is formed when the phase difference between fluorescence intensity and R-wave is not constant, will be described. When a cardiomyocyte injury progresses, the cardiomyocyte fails to conduct electricity, and thus the cardiomyocyte fails to contract by itself at time of heartbeat. An electric-conduction block, which is formed by the injured cardiomyocytes and has a box shape, fails to contract by itself, and moves such that the electric-conduction block follows the contraction movement of the adjacent cardiac-muscle tissue. As a result, the contact state of the tip portion of the laser catheter 300 is unstable and changes every second. As a result, the phase difference between fluorescence intensity and R-wave becomes unstable.
In other words, the correlation between fluorescence intensity and R-wave moves backward and forward between the correlation shown in Fig. 15 and the correlation shown in Fig. 16.

In view of this, according to the electric-conduction-block-formation determining operation of this embodiment, it is possible to determine that an electric-conduction block is formed in real time based on the phase difference between fluorescence intensity and electrocardiogram R-wave.
Specifically, in the case where the peak of fluorescence intensity appears for a predetermined time period after appearance of R-wave, it can be determined that an electric-conduction block is yet to be formed, and that the tip portion of the laser catheter 300 is in the upright-contact state. In the case where the peak of fluorescence intensity and the R-wave appear substantially simultaneously, it can be determined that an electric-conduction block is yet to be formed, and that the tip portion of the the laser catheter 300 is in the slanting-contact state. In the case where the phase difference between the peak of fluorescence intensity and R-wave is not constant, it can be determined that an electric-conduction block is formed.

Note that, because it can be determined that an electric-conduction block is formed in the case where the phase difference between fluorescence intensity and R-wave is not constant, if the display unit 170 displays the correlation between fluorescence intensity and R-wave on the display screen, a practitioner may estimate whether an electric-conduction block is formed or not based on the correlation between fluorescence intensity and R-wave, even if the controller 150 does not estimate whether an electric-conduction block is formed or not.

### <Second Embodiment>

Next, a PDT apparatus according to another embodiment of the present invention will be described. In the following description, descriptions of configurations, functions, operations, and the like similar to those of the PDT apparatus 1 of the first embodiment will be omitted or simplified, and different points will mainly be described.
An optical system and a detection unit according to the second embodiment will be described.

### [Structures of optical system and detection unit]

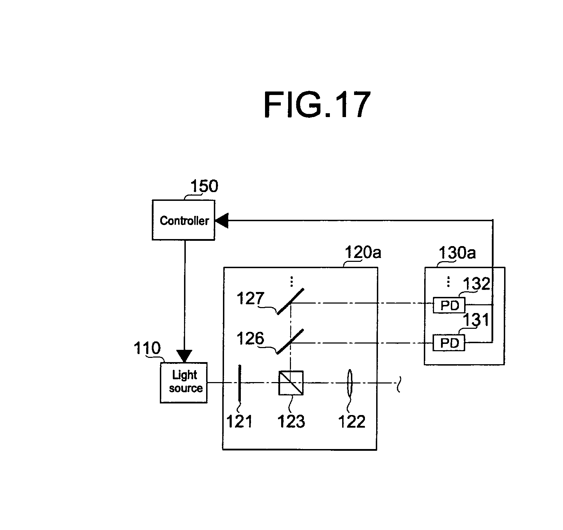

Fig. 17 is a block diagram showing an optical system, a detection unit, and the like of the second embodiment of the present invention.
An optical system 120a includes the short pass filter 121, the first lens 122, the PBS 123, a first dichroic mirror (hereinafter referred to as "DM".) 126, and a second DM 127.

A detection unit 130a includes a first photodiode (hereinafter referred to as "PD".) 131, and a second PD 132.

The first DM 126 reflects light having a certain wavelength out of the light entered from the PBS 123, and causes the light having the other wavelengths to pass through. In this manner, the first DM 126 reflects part of fluorescence from the laser catheter 300, and allows the fluorescence having the other wavelengths from the laser catheter 300 and specular reflection light to pass thorough. The fluorescence, which has reflected off the first DM 126, enters the first PD 131.
The first PD 131 detects the fluorescence entered from the first DM 126. The first PD 131 outputs the detected fluorescence intensity to the controller 150 as an electrical signal.

The second DM 127 reflects light having a certain wavelength out of the light which has passed through the first DM 126, and causes the light having the other wavelengths to pass through. In this manner, the second DM 127 reflects part of fluorescence, which has passed through the first DM 126, and allows the fluorescence having the other wavelengths and specular reflection light to pass thorough. The fluorescence, which has reflected off the second DM 127, enters the second PD 132.
The second PD 132 detects the fluorescence entered from the second DM 127. The second PD 132 outputs the detected fluorescence intensity to the controller 150 as an electrical signal.

Note that the optical system 120a may further include DMs each having a structure similar to the structure of each of the first DM 126 and the second DM 127. In this manner, eventually, the plurality of DMs 126, 127 ... reflect the fluorescence from the laser catheter 300, and the plurality of PDs 131, 132 ... detect the fluorescence from the laser catheter 300. Then, the plurality of DMs 126, 127 ... causes the specular reflection light to pass through.

Note that, as another embodiment, a pulse light source may be used as the light source 110, and the specular reflection light reflected off the fiber entrance edge may be temporally separated based on optical path length difference (about twice as long as length of laser catheter 300).

### <Third Embodiment>

In the third embodiment, the contact-monitoring steps are performed based on the phase difference between the peak of fluorescence intensity, which is in the interval between R-waves, and R-wave.
In the electric-conduction-block-formation determining operation of the first embodiment, it is determined that an electric-conduction block is formed based on the phase difference between the peak of fluorescence intensity, which is in the interval between R-waves, and R-wave. This principle may be applied to the contact-monitoring operation.

The light source 110 outputs the excitation light with the first intensity to the optical system 120. The detection unit 130 detects the fluorescence entered from the optical system 120. The detection unit 130 outputs the detected fluorescence intensity to the controller 150 as an electrical signal. The controller 150 calculates fluorescence intensity based on the obtained electrical signal.

Meanwhile, the electrocardiograph 140 obtains an electrocardiographic signal, and supplies the obtained electrocardiographic signal to the controller 150. The controller 150 creates display information based on the calculated fluorescence intensity and the obtained electrocardiographic signal, and outputs display instruction including the created display information to the display unit 170. The display unit 170 obtains the display instruction from the controller 150, and displays the correlation between fluorescence intensity and electrocardiogram R-wave on the display screen based on the display information in the display instruction.

The controller 150 determines the contact state of the tip portion of the laser catheter 300 based on the calculated fluorescence intensity and the obtained electrocardiographic signal (Step S106). Specifically, if the controller 150 determines that the peak of fluorescence intensity appears for a predetermined time period after appearance of the R-wave, the controller 150 determines that the tip portion of the laser catheter 300 is in the upright-contact state. If the controller 150 determines that the peak of fluorescence intensity and the R-wave appear simultaneously, the controller 150 determines that the tip portion of the laser catheter 300 is in the slanting-contact state. Further, the blood volume between the tip portion of the laser catheter 300 and the inner wall of a tissue may be estimated based on fluorescence peak intensity.

### <Fourth Embodiment>

According to the fourth embodiment, the contact-monitoring steps are performed by using autofluorescence spectrum differences. Note that, autofluorescence indicates light emitted from a tissue by itself, and does not mean fluorescence from a pharmaceutical. That is, the fourth embodiment describes a diagnostic method in which no pharmaceutical is used.

The light source 110 outputs an excitation light, with which the difference between the autofluorescence spectrum property of a cardiac-muscle tissue and the autofluorescence spectrum property of blood is determined easily. The detection unit 130 detects entered fluorescence. The detection unit 130 outputs the detected fluorescence intensity to the controller 150 as an electrical signal. The controller 150 calculates the fluorescence spectrum based on the obtained electrical signal. The controller 150 determines whether the calculated fluorescence spectrum shows the autofluorescence spectrum property of a cardiac-muscle tissue or the autofluorescence spectrum property of blood. The controller 150 compares the calculated fluorescence spectrum with the autofluorescence spectrum property of a cardiac-muscle tissue and the autofluorescence spectrum property of blood, and determines the contact state of the tip portion of the laser catheter 300 (Step S106). Specifically, if the controller 150 determines that the calculated fluorescence spectrum shows the autofluorescence spectrum property of a cardiac-muscle tissue, the controller 150 determines that the tip portion of the laser catheter 300 is in the upright-contact state. If the controller 150 determines that the calculated fluorescence spectrum shows the autofluorescence spectrum property of blood, the controller 150 determines that the tip portion of the laser catheter 300 is in the non-contact state. If the controller 150 determines that the calculated fluorescence spectrum does not show the each autofluorescence spectrum property, the controller 150 determines that the tip portion of the laser catheter 300 is in the slanting-contact state.

The contact-monitoring using the autofluorescence spectrum difference is also useful for laser-catheter contact-monitoring in the case of a therapy for a disease with a vascular occlusion (for example, arteriosclerotic disease or the like).
Figs. 18 are schematic diagrams each showing a contact state of a laser catheter in an intravascular lumen.
In a therapy for a disease with a vascular occlusion, it is desired to determine whether the tip portion of the laser catheter 300 contacts a vascular occlusion (atheromatous plaque) 21 in a blood vessel 20 (see Fig. 18(a)) or contacts a blood-vessel wall 22 (see Fig. 18(b)). Here, the composition ratio of collagen, elastin, lipid, and the like of a vascular occlusion is different from the composition ratio of a blood-vessel wall. Specifically, the composition ratio of a vascular occlusion (arteriosclerosis) is 70% of water, 5% of collagen, 6% of elastin, and 9% of lipid. The composition ratio of a blood vessel is 73% of water, 6.5% of collagen, 10.5% of elastin, and 1% of lipid. Because of this, the autofluorescence spectrum property of a vascular occlusion is different from the autofluorescence spectrum property of a blood-vessel wall. If a therapy-target site is irradiated with an excitation light, with which the property difference is determined easily, and fluorescence is measured, it is possible to determine whether the tip portion of the laser catheter 300 contacts the vascular occlusion 21 or the blood-vessel wall 22. Note that, because it can be determined whether there is an atheromatous plaque or not based on the composition ratio, the contact-monitoring using the autofluorescence spectrum difference can perform diagnosis more precisely than IVUS (intravascular ultrasound), with which it is determined whether there is an atheromatous plaque or not based on the size of the blood vessel diameter.

The embodiments of the present invention are not limited to the above-mentioned embodiments, and other various embodiments are conceivable.

Although in the above-mentioned embodiments, the laser catheter 300 is detachably connected to the connector 210 of the PDT apparatus 1, the laser catheter 300 may be provided on the PDT apparatus 1 integrally.
Although in the above-mentioned embodiments, the tube 200 is provided on the PDT apparatus main body 100 and the connector 210 is provided on the end of the tube 200, the connector 210 may be provided on the PDT apparatus main body 100.
Although in the above-mentioned embodiments, the PBS 123 is used, a DM may be used instead.

Although in the above-mentioned embodiments, the controller 150 informs a practitioner of information to prompt the predetermined controls by using the display unit 170, but is not limited to this. A speaker unit may be provided on the PDT apparatus 1, and the controller 150 may create a sound output instruction when prompting a practitioner to perform the predetermined controls, may output the created sound output instruction to the speaker unit, and may cause the speaker unit to output sounds, to thereby prompt a practitioner to perform the predetermined controls. Description of Symbols

- 1: photodynamic therapy (PDT) apparatus
- 100: PDT apparatus main body
- 110: light source
- 120, 120a: optical system
- 121: short pass filter
- 122: first lens
- 123: polarizing beam splitter (PBS)
- 124: long pass filter
- 125: second lens
- 126: first dichroic mirror (DM)
- 127: second dichroic mirror (DM)
- 130, 130a: detection unit
- 131: first photodiode (PD)
- 132: second photodiode (PD)
- 140: electrocardiograph
- 141: electrode pad
- 150: controller
- 160: storage
- 170: display unit
- 180: controller
- 200: tube
- 201: apparatus-attached optical fiber
- 210: connector
- 300: laser catheter
- 301: irradiation light
- 310: catheter tube
- 320: holder
- 330: optical fiber
- 340: optical window

## Claims

1. A calculation apparatus for irradiating a tissue having absorbed photo-sensitive pharmaceutical, the photo-sensitive pharmaceutical absorbing an excitation light and emitting fluorescence, with the excitation light emitted from a tip portion of a laser catheter, comprising:
a connector to/from which the laser catheter is capable of being attached/detached;
a light source for outputting the excitation light to the laser catheter via the connector; and
a detection unit for detecting intensity of the fluorescence, the fluorescence being entered from the laser catheter via the connector, to calculate concentration of the photo-sensitive pharmaceutical in a tissue, the tip portion of the laser catheter contacting the tissue.

2. The calculation apparatus according to claim 1, further comprising:
a controller for calculating the concentration of the photo-sensitive pharmaceutical in the tissue, the tip portion of the laser catheter contacting the tissue, based on intensity of the detected fluorescence.

3. The calculation apparatus according to claim 2, wherein
the controller outputs a signal to prompt to additionally administer the photo-sensitive pharmaceutical based on the calculated concentration.

4. The calculation apparatus according to claim 2, wherein
the controller calculates an excitation-light-irradiation protocol based on the calculated concentration, and outputs a calculation result.

5. A calculation method, comprising:
irradiating a tissue having absorbed photo-sensitive pharmaceutical, the photo-sensitive pharmaceutical absorbing an excitation light and emitting fluorescence, with the excitation light emitted from a tip portion of a laser catheter;
extracting the fluorescence corresponding to the irradiated excitation light via the laser catheter; and
calculating concentration of the photo-sensitive pharmaceutical in a tissue, the tip portion of the laser catheter contacting the tissue, based on intensity of the extracted fluorescence.

6. The calculation method according to claim 5, further comprising:
calculating an excitation-light-irradiation protocol based on the calculated concentration, and outputs a calculation result.

7. A calculation method using photo-sensitive pharmaceutical absorbing an excitation light and emitting a fluorescence, a laser catheter capable of emitting the excitation light from a tip portion, and a calculation apparatus including a connector to/from which the laser catheter is capable of being attached/detached and a light source for outputting the excitation light to the laser catheter via the connector, comprising:
absorbing, in a tissue, the photo-sensitive pharmaceutical;
leading the tip portion of the laser catheter to the tissue having absorbed the photo-sensitive pharmaceutical, the laser catheter being attached to the connector;
irradiating the tissue having absorbed the photo-sensitive pharmaceutical with the excitation light emitted from the tip portion of the laser catheter, the excitation light being output from the light source;
extracting the fluorescence corresponding to the irradiated excitation light via the laser catheter; and
calculating concentration of the photo-sensitive pharmaceutical in a tissue, the tip portion of the laser catheter contacting the tissue, based on intensity of the extracted fluorescence.

8. The calculation method according to claim 7, further comprising:
calculating an excitation-light-irradiation protocol based on the calculated concentration, and outputs a calculation result.
